# EUROPEAN PATENT APPLICATION

(11) **EP 2 674 089 A1**
(43) Date of publication of application: **18.12.2013**
(21) Application number: 12844530.1
(22) Date of filing: 26.09.2012
(51) Int. Cl.: A47L 11/34, A61L 2/07

(54) **TABLE CLEANER**

(30) Priority: 24.10.2011 KR 20110108436; 17.09.2012 KR 20120102800
(71) Applicant: Kyoryu Electric Co., Ltd., Incheon 407-040 (KR)
(72) Inventor: KIM, Yongoh, Seoul 157-030 (KR)
(74) Representative: Romano, Giuseppe
(86) International application number: PCT/KR2012/007702
(87) International publication number: WO 2013/062241

(57) **Abstract**

A table cleaner includes a main body case; a storage battery disposed on an upper portion of the main body case and configured to store power supplied from outside; a steam generation unit disposed inside the main body case and configured to generate and supply steam by heating water injected through a lower portion spaced from the storage battery by using power supplied from the storage battery; and an ejection cleaning unit disposed on a lower portion of the main body case and configured to clean a table surface by ejecting steam, which is generated by the steam generation unit, in a downward direction.

## Description

### Technical Field

The present invention relates to a table cleaner, and more particularly, to a table cleaner for cleaning a table using steam, which is ejected while blocking moisture by spacing a location of heating for steam generation from a location of steam ejection, thereby improving steam generation efficiency and safety.

### Background Art

In general, a table is cleaned by applying a cleaning liquid, such as detergent, and manually cleaning the table. However, a table, which is used when a user eats or prepares food, is easily contaminated by food scraps, cooking by-products, food fragments scattered during eating, and the like, and, when exposed to air for a long period of time, the organic contaminants decay and hazardous microorganisms multiply. The multiplied microorganisms, if introduced into human bodies via silverwares or through direct contact with users, may cause diseases. Furthermore, when a table is contaminated by food containing moisture, the food adheres to the table as the moisture evaporates, making it difficult to remove. In addition, such a conventional table cleaning method cannot easily remove stains, which develop if the table has not been cleaned for a long period of time, or which develop due to contamination by oil.

Such a conventional table cleaning method has a problem in that the table needs to be cleaned manually and repeatedly using a large amount of detergent, requiring a long cleaning time and causing a problem of re-contamination by the detergent.

Furthermore, a cleaning material (for example, rag) used for manual cleaning operations may cause microorganisms to multiply, if it is not sterilized frequently after being contaminated during table cleaning, and may re-contaminate tables.

Therefore, table cleaners have been developed and used, which boil water and eject the resulting steam of vapor form to clean and sterilize tables at the same time.

However, such a conventional table cleaner has a problem in that a heating member, which receives power and generates heat, is provided in the direction of steam ejection, meaning that moisture may flow into the power supply location and shorten the service life. There is also an increasing danger that short circuit or leakage of electricity may occur and cause fire.

In addition, a conventional table cleaner, which ejects high-temperature steam for cleaning and sterilizing, has a problem as follows: in the case of poor control of the direction, speed, and amount of ejected high-temperature steam, it may be directed to the user and cause an injury (for example, scald). In addition, high-temperature water is ejected together with steam, requiring a long drying time.

### SUMMARY OF INVENTION

### Technical Problem

The present invention has been made in an effort to solve the above-mentioned problems, and it is an object of the present invention to provide a table cleaner for cleaning a table using steam, which is ejected while blocking moisture by spacing a location of heating for steam generation from a location of steam ejection, thereby improving steam generation efficiency and safety.

The technical object of the present invention is not limited to the above-mentioned ones, and other undisclosed technical objectives will be clearly understood from the following description by those skilled in the art.

### Solution to Problem

In order to accomplish the above-mentioned object, a table cleaner according to an embodiment of the present invention includes: a main body case; a storage battery disposed on an upper portion of the main body case and configured to accumulate power supplied from outside; a steam generation unit disposed inside the main body case and configured to generate and supply steam by heating water injected through a lower portion spaced from the storage battery by using power supplied from the storage battery; and an ejection cleaning unit disposed on a lower portion of the main body case and configured to clean a table surface by ejecting steam, which is generated by the steam generation unit, in a downward direction.

The steam generation unit may include: a steam generation member disposed inside the main body case and provided with a steam generation space having an opened lower portion; a heating member disposed on an upper portion of the steam generation member and configured to generate steam inside the steam generation member by generating heat using power supplied from the storage battery; a steam valve disposed on the upper portion of the steam generation member and configured to communicate with the steam generation space and supply the ejection cleaning unit with steam generated in the steam generation space; a steam sealing member coupled to a lower portion of the steam generation member to seal the steam generation space having the opened lower portion, the steam sealing member having a water injection member at a center, the water injection member protruding to have a water injection hole through which water is injected, the steam sealing member having an injection fastening portion formed inside the water injection hole so that the ejection cleaning unit is fastened to the injection fastening portion, the steam sealing member having a steam injection member having a steam injection channel formed inside to communicate with the water injection hole so that steam, which is injected from the steam valve through one side of the steam generation member, is supplied to the water injection hole; and a steam connection member disposed on one side of the steam sealing member, one end of the steam connection member being connected to the steam valve through a steam supply tube, the other end being connected to the steam injection channel so that steam, which is injected from the steam valve, is supplied to the ejection cleaning unit.

The steam generation unit may further include: generation fastening protrusions disposed at a plurality of locations on an outer periphery of the steam generation member, the generation fastening protrusion each having a generation fastening hole formed inside in a through-hole form; sealing fastening protrusions protruding upwards from a plurality of locations on the steam sealing member while being positioned to contact and fasten to the generation fastening protrusions, respectively; and sealing fastening members inserted into the generation fastening holes to fasten the generation fastening protrusions and the sealing fastening protrusions and couple the steam generation member and the steam sealing member.

The steam generation unit may have a generation fastening portion formed on an inner surface of a lower portion of the steam generation member, the steam sealing member being fastened to the generation fastening portion, and a sealing fastening portion may be formed on an outer periphery of the steam sealing member, the generation fastening portion being fastened to the sealing fastening portion, so that the steam generation member and the steam sealing member are coupled.

The ejection cleaning unit may include: an ejection member disposed on a lower portion of the steam generation unit, water being injected through a center of the ejection member, an ejection fastening portion being formed on an outer surface of the ejection member to be fastened to the steam generation unit, and an ejection channel being formed through a lateral surface connected to the steam generation unit so that steam is supplied downwards; a cleaning member disposed on a lower portion of the ejection member, an ejection hole being formed through a center of the cleaning member to communicate with the ejection channel so that steam is ejected, a cleaning recess being formed on a lower surface of the cleaning member; and a cleaning material disposed on the lower surface of the cleaning member and inserted into the cleaning recess to contact the table surface, and the ejection member is, when unfastened from the steam generation unit, separated from the main body case so that water is injected into the steam generation unit, and the ejection member is, after injection is finished, fastened to the steam generation unit by the ejection fastening portion so that steam generated by the steam generation unit can be ejected through the ejection hole via the ejection channel.

The steam sealing member and the ejection member may be integrally formed.

The table cleaner may further include a support charging unit, on which the lower portion of the main body case is seated, or to which the storage battery is coupled and charged.

A charging connection recess may be formed at a center of an upper portion of the storage battery, the support charging unit being inserted and connected to the charging connection recess to supply power. The support charging unit may include: a support member disposed on one side of the main body case, a support space being formed inside the support member, the main body case being seated in the support space, a support latch being formed towards an upper portion of the support space, the ejection cleaning unit being engaged and supported on the support latch; a charging protrusion protruding upwards from a center of an inner portion of the support member, the charging protrusion being positioned upwards at a center of the support space so that the main body case is inverted, with the storage battery facing downwards, and is inserted into the charging connection recess and charged; and a power supply switch disposed on one side of the support member and configured to be turned on/off by external manipulation to control power supply to the charging protrusion, and the support member may be configured so that, when the main body case is seated during use, the ejection cleaning unit remains engaged and supported on the support latch, and, during charging, the main body case is inverted and inserted into the support space to enable the storage battery to be inserted into and supported on the charging protrusion and charged.

The table cleaner may further include a pressure regulation unit disposed between the steam generation space and an ejection location of the ejection cleaning unit and configured to open the ejection cleaning unit and ejects steam to outside, when steam is generated by heating of the heating member and when internal pressure of the steam generation space exceeds a preset threshold, and seal the ejection cleaning unit when the preset threshold is not exceeded.

The pressure regulation unit may include: a regulation member disposed inside the ejection cleaning unit, the regulation member having a regulation space having one side open in a steam ejection direction of the ejection cleaning unit and the other side sealed towards the steam generation unit, the regulation member having a channel connection hole formed on a lateral surface to communicate so that steam generated by the steam generation unit is supplied, the regulation member having a steam inflow hole formed at a center of the sealed other side of the regulation space to communicate with the steam generation unit so that heated steam flows in, and the regulation member having a regulation fastening portion formed on an outer periphery to be fastened to the ejection cleaning unit; a regulation opening/closing member disposed inside the regulation member and installed inside the regulation space between a location of one side of the steam inflow hole and a steam ejection location of the ejection cleaning unit so as to open/close the steam inflow hole and the steam ejection location of the ejection cleaning unit; and an operation elastic member disposed between the ejection cleaning unit and the regulation opening/closing member and installed to provide elastic force in such a direction that the regulation opening/closing member seals the steam inflow hole.

Details of other embodiments are included in the following description and the accompanying drawings.

### Advantageous Effects of Invention

The table cleaner according to an embodiment of the present invention cleans a table using steam, which is ejected while blocking moisture by spacing a location of heating for steam generation from a location of steam ejection, thereby preventing inflow of moisture into the power supply location. This prevents reduction of service life due to contact with moisture, as well as short circuit and leakage of electricity, which could otherwise cause fire and blackout. Therefore, the service life is extended, and safety is improved.

Furthermore, the location where steam and water of the table cleaner are supplied is spaced from the location of power supply and heating so that water supply and power supply are conducted in different directions. Therefore, steam generation and steam ejection are conducted respectively while switching the direction physically, enabling effective injection of water and effective steam generation in a sealed condition after direction switching.

The table cleaner is installed on the support means in inversed conditions between when charged for steam generation and when held during steam ejection use, so that the user can easily recognize whether it is being charged or held, thereby improving user convenience.

In addition, steam of the table cleaner is generated, its pressure is regulated by the steam valve, and the steam is ejected and supplied. In this state, the range of ejection of the high-temperature steam is maintained constantly when a table is cleaned by the cleaning material. This prevents scattering of steam due to excessive pressure, which could otherwise cause scald, for improved safety and also prevents degradation of cleaning and sterilization efficiency due to insufficient pressure.

Moreover, the table cleaner includes a pressure regulation unit configured to regulate steam pressure at the location of steam ejection and control the pressure in a twofold manner together with the steam valve. This maintains the range of ejection of high-temperature steam constantly, prevents scattering of steam due to excessive pressure, which could otherwise cause scald, and improves safety.

Advantageous effects of the present invention are not limited to those mentioned above, and others undisclosed can be clearly understood from the accompanying claims.

### Brief Description of Drawings

Fig. 1 is a perspective view illustrating a table cleaner according to an embodiment of the present invention.
Fig. 2 is a front view illustrating the table cleaner of Fig. 1.
Fig. 3 is an exploded perspective view illustrating major parts of the table cleaner of Fig. 1.
Fig. 4 is a sectional view illustrating major parts of the table cleaner of Fig. 1.
Fig. 5 is a use state diagram illustrating the table cleaner of Fig. 1, which is separated before holding.
Fig. 6 is a use state diagram illustrating the table cleaner of Fig. 1, which is being charged.
Fig. 7 is a use state diagram illustrating the table cleaner of Fig. 1, which is separated after charging.
Fig. 8 is a front view illustrating the table cleaner of Fig. 1, which is being charged.
Fig. 9 is a partially exploded perspective view illustrating major parts of the table cleaner of Fig. 1.
Fig. 10 is a sectional perspective view illustrating major parts of the table cleaner of Fig. 9.
Fig. 11 is a sectional view illustrating a table cleaner according to another embodiment of the present invention.
Fig. 12 is a sectional view illustrating a table cleaner according to still another embodiment of the present invention.

### Description of Embodiments

Hereinafter, exemplary embodiments of the present invention will be described in detail with reference to the accompanying drawings so that those skilled in the art to which the present invention pertains can easily practice the present invention.

In the following description of embodiments, technical details, which are well known in the technical field of the present invention and have no direct relevance to the present invention, will not be described. This is for the purpose of clarifying the essentials of the present invention, not obscuring them, by omitting unnecessary descriptions.

For similar reasons, some components in the accompanying drawings are exaggerated, omitted, or illustrated schematically. Also, the size of each component does not necessary reflect the actual size. The same reference numerals are assigned to identical or relevant components throughout the drawings.

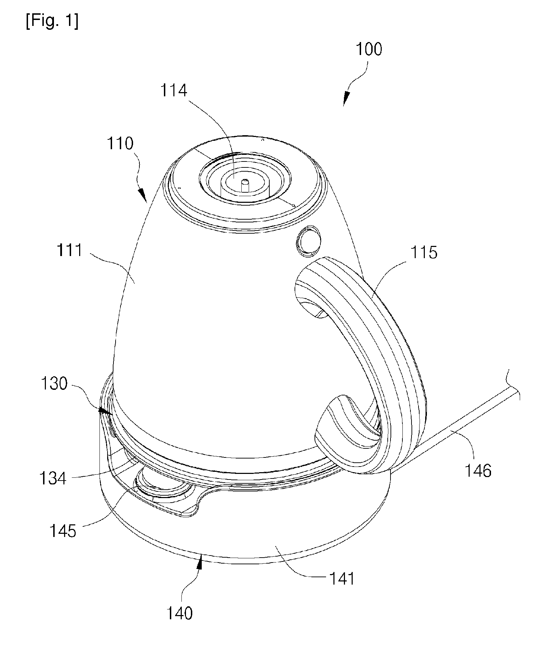

Hereinafter, a table cleaner according to an embodiment of the present invention will be described with reference to Figs. 1 to 10.

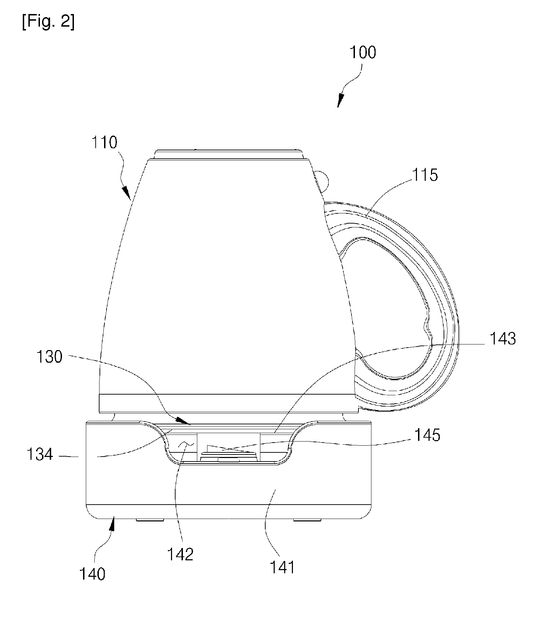

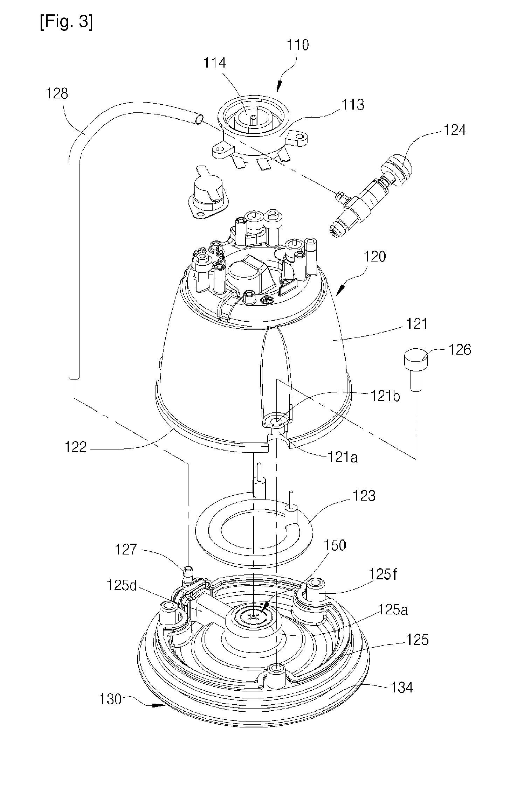

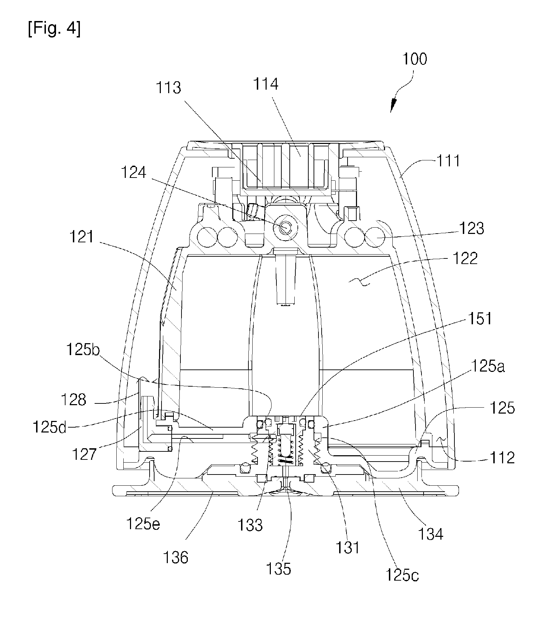

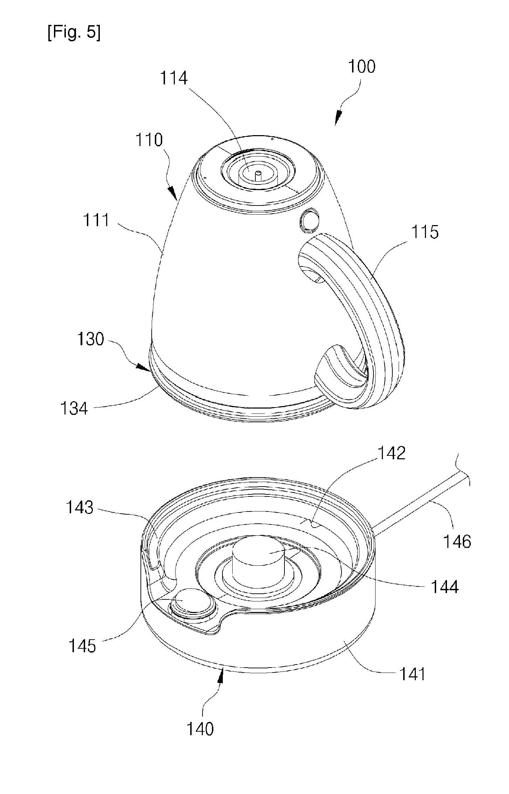

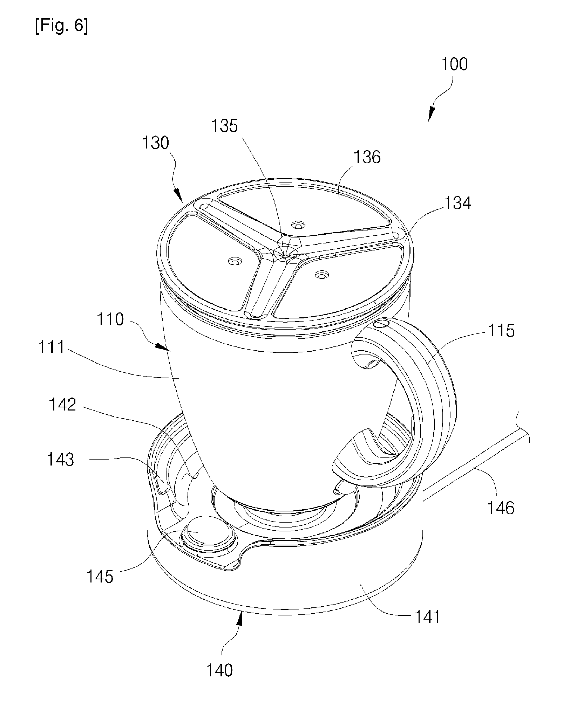

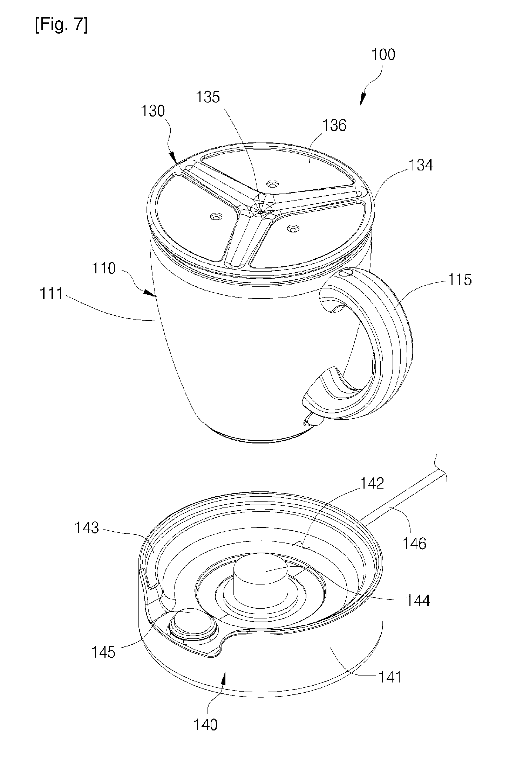

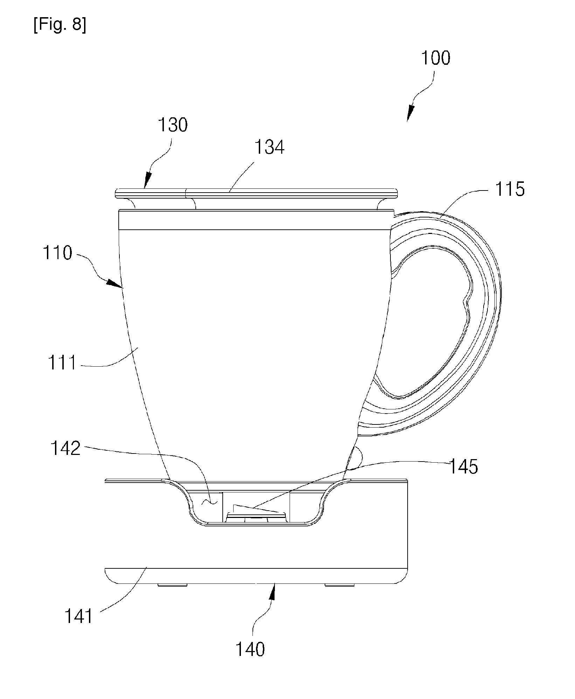

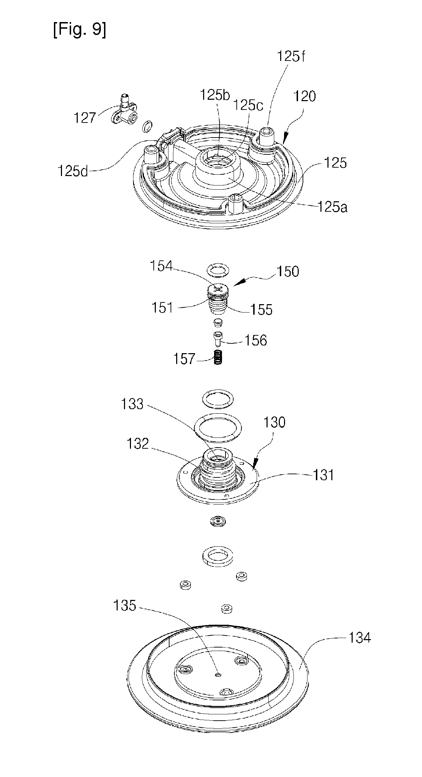

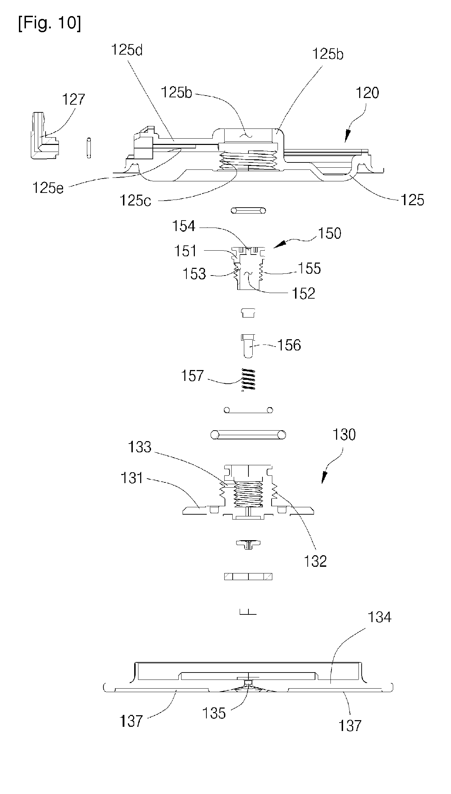

Fig. 1 is a perspective view illustrating a table cleaner according to an embodiment of the present invention; Fig. 2 is a front view illustrating the table cleaner of Fig. 1; Fig. 3 is an exploded perspective view illustrating major parts of the table cleaner of Fig. 1; Fig. 4 is a sectional view illustrating major parts of the table cleaner of Fig. 1; Fig. 5 is a use state diagram illustrating the table cleaner of Fig. 1, which is separated before holding; Fig. 6 is a use state diagram illustrating the table cleaner of Fig. 1, which is being charged; Fig. 7 is a use state diagram illustrating the table cleaner of Fig. 1, which is separated after charging; Fig. 8 is a front view illustrating the table cleaner of Fig. 1, which is being charged; Fig. 9 is a partially exploded perspective view illustrating major parts of the table cleaner of Fig. 1; and Fig. 10 is a sectional perspective view illustrating major parts of the table cleaner of Fig. 9.

Referring to Figs. 1 to 10, a table cleaner 100 according to an embodiment of the present invention includes a main body 110, a steam generation unit 120, an ejection cleaning unit 130, a support charging unit 140, and a pressure regulation unit 150. The main body 110 includes a main body case 111, on which various components of the table cleaner 100 is mounted, a storage battery 113, and a main body handle 115. The main body case 111 has an opening 112 formed on its lower end, through which the steam generation unit 120 and the ejection cleaning unit 130 are installed on the inner and lower portions, respectively.

The storage battery 113 is disposed on the upper portion of the main body case 111 and is installed to accumulate and store power, which is supplied from the outside, and then supply the steam generation unit 120 with the power so that steam is generated. The storage battery 113 is installed to accumulate the supplied power, store the power while being moved, and supply the steam generation unit 120 with the power so that steam is generated.

The storage battery 113 has a charging connection recess 114 formed at the center of its upper portion in a recess form to be inserted and connected to the support charging unit 140 to supply power. The charging connection recess 114 is provided in a recess form to be inserted into the support charging unit 140 and supply the storage battery 113 with the power.

The main body handle 115 is fixed to a lateral surface of the main body case 111 and is provided in a handle form so that the user can grasp it on a lateral surface of the main body case 111. The main body handle 115 is installed on a lateral surface so that the user can clean a table while easily grasping the main body case 111, which is heated due to steam generation.

The steam generation unit 120 includes a steam generation member 121 configured to generate steam inside the main body case 111, a heating member 123, a steam valve 124, a steam sealing member 125, sealing fastening members 126, a steam connection member 127, and a steam supply tube 128.

The steam generation member 121 is disposed inside the main body case 111 and is configured to generate steam by means of heat from the heating member 123. A steam generation space 122 is formed inside the steam generation member 121 with its lower portion open downwards. The steam sealing member 125 is installed on the opened lower portion of the steam generation space 122 to seal the steam generation space 122 and generate steam.

Generation fastening protrusions 121a are formed on the outer periphery of the steam generation member 121 so as to protrude outwards at a plurality of locations, and generation fastening holes 121b are formed inside the generation fastening protrusions 121a in a through-hole form. The generation fastening holes 121b are configured so that sealing fastening members 126, which are used to seal the steam generation space 122 by the steam sealing member 125, are inserted therein to fasten and fix the steam generation member 121 and the steam sealing member 125 to each other.

The heating member 123 is disposed on the upper portion of the steam generation member 121 and is connected to the storage battery 113 so that it generates heat using supplied power and thereby generates steam inside the steam generation member 121. The heating member 123 is installed on the upper portion of the steam generation member 121 to receive power from the connected storage battery 113 and generate heat so that water, which is contained in the steam generation space 122, turns into steam by means of the generated heat. Although the heating member 123 is configured in a coil form in the present embodiment, the present invention is not limited thereto. That is, it is obvious to those skilled in the art that the heating member 123 includes any means for receiving power and generating heat. The heating member 123 is disposed on the upper portion of the main body case 111 adjacent to the storage battery 113, thereby improving power supply efficiency. In addition, the heating member 123, which generates heat using power supplied from the storage battery 113, is installed at a location on the upper portion which is spaced from the lower portion, where the steam sealing member 125, into which water is injected, and the ejection cleaning unit 130, which ejects steam, are installed, thereby preventing inflow of moisture into locations of power supply and heat generation. This prevents short circuit, leakage of electricity, discharge, and the like, which could occur if power and moisture contact, thereby improving safety and increasing the service life.

The steam valve 124 is disposed on one side of the upper portion of the steam generation member 121 and is installed at a location, where it communicates with one side of the upper portion of the steam generation space 122, so as to supply the ejection cleaning unit 130 with generated steam while controlling its pressure.

The steam valve 124 is disposed on one side of the upper portion where it can supply steam from the steam generation member 121 to the ejection cleaning unit 130 and is installed to communicate with the steam generation space 122 and control the pressure of steam generated therein. If steam generated inside the steam generation space 122 is ejected at an excessive pressure without being controlled, that is, if steam is ejected from the ejection cleaning unit 130 at a high pressure and is scattered over a larger area and at higher intensity than expected by the user, the danger of scald increases. If steam is ejected at a low pressure without being controlled, high-temperature water is ejected together with the steam and degrades cleaning efficiency. Therefore, a steam valve 124 is installed to eject steam under a constant-pressure atmosphere, which improves safety and cleaning efficiency. That is, the steam valve 124 regulates the pressure of steam generated by the steam generation member 121 and supplies steam ejected at a pressure that enables cleaning, thereby improving supply efficiency.

The steam sealing member 125 is disposed on the lower portion of the steam generation member 121 and is installed to seal the steam generation space 122, the lower portion of which is open. A water injection member 125a is provided at the center of the steam sealing member 125 and protrudes so that a water injection hole 125b is formed through to enable water injection into the steam generation space 122. The water injection hole 125b is formed in the form of a through-hole to enable water to be injected into the steam generation space 122 through the center of the lower surface, which is spaced from the storage battery 113 positioned on the upper portion of the steam generation member 121 to accumulate and supply power. That is, a water injection hole 125b is formed on the lower portion of the steam generation member 121 to enable water injection so that inflow of moisture into the storage battery 113 is prevented during water injection. This increases the service life of the storage battery 113 and prevents short circuit and leakage of electricity due to inflow of moisture into the storage battery 113.

An injection fastening portion 125c is formed on the inner surface of the water injection hole 125b to be fastened to the ejection cleaning unit 130. The water injection hole 125b is sealed when the ejection cleaning unit 130 is fastened to the injection fastening portion 125c and is opened when the ejection cleaning unit 130 is unfastened, then allowing water injection into the steam generation space 122. That is, the steam generation member 121 installed inside the main body case 111 is configured so that, when the ejection cleaning unit 130 is unfastened from the water injection hole 125b during water injection into the steam generation space 122, the water injection hole 125b is opened. After the water injection hole 125b is opened, water is injected in an inverted condition, in which the water injection hole 125b is positioned upwards. After water injection, the ejection cleaning unit 130 is fastened to the injection fastening portion 125c to guarantee sealing, and the heating member 123 is then used to generate steam through heating.

A steam injection member 125d is provided to protrude from one side of the steam sealing member 125 towards the center, and a steam injection channel 125e is formed therein to communicate with the water injection hole 125b so that steam, which is injected from the steam valve 124 through one side of the steam generation member 121, is supplied to the water injection hole 125b.

Sealing fastening protrusions 125f protrude from a plurality of locations on the steam sealing member 125 so as to contact and fasten with the generation fastening protrusions 121 a.

Sealing fastening members 126 are provided on the outer periphery of the steam generation member 121 and are configured to be inserted into the generation fastening holes 121b to fasten the generation fastening protrusions 121a and the sealing fastening protrusions 125f and thereby couple the steam generation member 121 and the steam sealing member 125.

The steam connection member 127 is disposed on one side of the steam sealing member 125, one end of the steam connection member 127 is connected to the steam valve 124 via the steam supply tube 128, and the other end thereof is connected to the steam injection channel 125e, so that steam injected from the steam valve 124 is supplied to the ejection cleaning unit 130.

The steam supply tube 128 connects the steam valve 124 and the steam connection member 127, and is installed to supply steam, which is generated at the steam generation member 121, to the steam connection member 127, which supplies it to the ejection cleaning unit 130, via the steam valve 124. The steam supply tube 128 makes a connection so that steam, which is generated at the steam generation member 121, passes through the steam valve 124, which regulates its pressure, and reaches the ejection cleaning unit 130.

The ejection cleaning unit 130 includes an ejection member 131 installed on the lower portion of the steam generation member 121 to be opened/closed, a cleaning member 134, and a cleaning material 137. The ejection member 131 is disposed on the lower portion of the center of the steam sealing member 125 and is installed to open/close the water injection hole 125b of the steam sealing member 125 so that steam is supplied downwards.

An ejection fastening portion 132 is formed on the outer surface of the ejection member 131 to be fastened to the injection fastening portion 125c.

An ejection channel 133 is formed inside the ejection member 131 to extend downwards from a lateral surface through the inside so that steam is supplied downwards. The ejection channel 133 is formed in the form of a channel connected to the steam injection channel 125e through the lateral surface so that supplied steam is ejected downwards through the inside. The ejection channel 133 is formed so that, while the ejection member 131 is fastened inside the water injection hole 123(→ 125b), steam generated in the steam generation space 122 is supplied through the steam injection channel 125e to the lower portion of the ejection member 131.

The cleaning member 134 is disposed on the lower portion of the ejection member 131 and is provided in the form of a flat plate having a size larger than the ejection member 131 so that the cleaning area is increased. The cleaning member 134 is positioned on the lower portion of the main body case 111 and is installed on the lower portion of the ejection member 131. An ejection hole 135 is formed through the center of the cleaning member 134 to communicate with the lower portion of the ejection channel 133. The ejection hole 135 is formed so that steam, which is ejected through the ejection channel 133, is directly ejected to a table to be cleaned. A plurality of cleaning recesses 136 are formed at locations on the lower surface of the cleaning member 134, where they can contact the table to be cleaned. The cleaning recesses 136 are formed so that a cleaning material 137, which is used to clean tables, is inserted and installed.

That is, the ejection member 131 is, when unfastened from the water injection hole 123(→ 125b), separated from the main body case 111 so that water is injected into the steam generation member 121; after injection is finished, the injection fastening portion 124(→ 125c) and the ejection fastening portion 132 are fastened to seal the steam generation member 121; the heating member 123 then emits heat and generates steam; the steam passes through the steam valve 124, which controls its pressure; and the steam supplied through the steam injection channel 125e passes through the ejection channel 133 and can be directly ejected through the ejection hole 135 to the table.

The cleaning material 137 is disposed on the lower surface of the cleaning member 134 and is inserted into the cleaning recesses 136 so that it contacts a table and cleans it. The cleaning material 137 is preferably a fibrous cleaning material capable of removing moisture, such as a rag or absorbent cloth, which is commonly used to clean tables. The cleaning material 137 can move while contacting a table, which is cleaned by directly ejected steam, and remove moisture.

The support charging unit 140 includes a support member 141 disposed on one side of the main body case 111, a charging protrusion 144, a power supply switch 145, and a power supply line 146. The support member 141 is disposed on one side of the main body case 111 and is installed so that the main body case 111 and the ejection cleaning unit 130 are seated and stored, and the main body case 111 is charged when seated. A support space 142 is formed inside the support member 141 so that the main body case 111 and the ejection cleaning unit 130 are seated therein. The support space 142 is formed so that, when the table cleaner 100 is seated after use, the ejection cleaning unit 130 is inserted and seated, and the storage battery 113 is inserted, during charging, with the main body case 111 inverted.

A support latch 143 is formed inside the support member 141 towards the upper portion of the support space 142 so as to engage and support the ejection cleaning unit 130 when it is seated. The support latch 143 is formed inside the support member 141 so that the ejection cleaning unit 130, which conducts cleaning when the table cleaner 100 is used, is engaged and supported to remain seated.

The charging protrusion 144 protrudes from the center of the inside of the support member 141 in the upward direction and is positioned at the center of the support space 142 in the upward direction so that it is inserted into the charging connection recess 114 to charge the storage battery 113 when the main body case 111 is inverted with the storage battery 113 facing downwards. The charging protrusion 144 is installed to be inserted into the charging connection recess 114, while being connected to the power supply switch 145 and the power supply line 146, to supply the storage battery 113 with power.

That is, the support member 141 is installed as follows: when the main body case 111 is seated during use, the ejection cleaning unit 130 remain engaged and supported on the support latch 143; during charging, the main body case 111 is inverted and inserted into the support space 142 so that the storage battery 113 is inserted and supported into the charging protrusion 144 and charged.

The power supply switch 145 is disposed on one side of the support member 141 and is turned on/off by external manipulation to control power supply to the charging protrusion 144. The power supply switch 145 is installed on one side of the support member 141 and is turned on/off by the user to control power supply to the storage battery 113, thereby manipulating the charging state.

The power supply line 146 is disposed on one side of the support member 141 and is installed to connect the charging protrusion 144 and a supply source (not illustrated), which supplies power, so that power is supplied. The power supply line 146 is installed to connect an external supply source, which supplies power, and the charging protrusion 144 so that power is supplied to the inserted storage battery 113.

The pressure regulation unit 150 includes a regulation member 151 installed towards the steam generation space 122 of the ejection member 131, a regulation opening/closing member 156, an operation elastic member 157, and an ejection opening/closing member 158.

The regulation member 151 is disposed inside towards the steam generation space 122 of the ejection member 131 and is installed at a location where steam is ejected. A regulation space 152 is formed inside the regulation member 151 so that it is open towards the ejection hole 135 of the cleaning member 134 while being sealed towards the steam generation space 122 of the steam generation member 121. A channel connection hole 153 is formed towards a lateral surface of the regulation member 151 to communicate with the regulation space 152 at a location where it is connected with the ejection channel 133. The channel connection hole 153 is connected to the ejection channel 133, to which steam generated in the steam generation space 122 is supplied, so that steam is ejected through the ejection hole 135. A steam inflow hole 154 is formed at the center of the sealed portion of the regulation space 152, which faces the steam generation space 122, so that the steam generation space 122 and the regulation space 152 communicate with each other and allow steam, which is generated in the steam generation space 122, to flow into the regulation space 152. A regulation fastening portion 155 is formed on the outer periphery of the regulation member 151 to be fastened to the ejection member 131. The regulation fastening portion 155 is provided in a screw form so that the regulation member 151 can be fastened to and separated from the ejection member 131.

The regulation opening/closing member 156 is disposed inside the regulation member 151 and is installed inside the regulation space 152 between the lower portion of the steam inflow hole 154 and the upper portion of the ejection hole 135 so as to open/close the steam inflow hole 154 and the ejection hole 135. The regulation opening/closing member 156 keeps sealing the steam inflow hole 154 and descends, when the pressure exceeds a preset threshold, to open the steam inflow hole 154 and seal the ejection hole 135, thereby preventing ejection of steam of excessive pressure through the ejection hole 135 and guaranteeing discharge at a lower pressure.

The operation elastic member 157 is disposed between the upper portion of the ejection hole 135 of the ejection channel 133 and the lower portion of the regulation opening/closing member 156 and is installed to provide elastic force in such a direction that the steam inflow hole 154 is sealed by the regulation opening/closing member 156. The operation elastic member 157 is installed to provide elastic force, which causes the regulation opening/closing member 156 to seal the steam inflow hole 154, and the sealing state of the regulation opening/closing member 156 is maintained during steam use and generation.

The heating member 123 continuously operates and generates steam, which flows in through the steam inflow hole 154 and pressurizes and lowers the regulation opening/closing member 156. When pressure generated by the steam exceeds a preset threshold and overcomes elastic force from the operation elastic member 157, the regulation opening/closing member 156 is lowered by the steam pressure and opens the steam inflow hole 154, thereby lowering pressure. This prevents explosion due to excessive pressure or leakage of steam and thus improves safety.

In addition, the fact that the regulation opening/closing member 156 descends and seals the ejection hole 135 prevents ejection of steam of excessive pressure, which is discharged to lower pressure, towards the ejection hole 135, thereby preventing any injury of the user resulting from ejection of stream of excessive pressure.

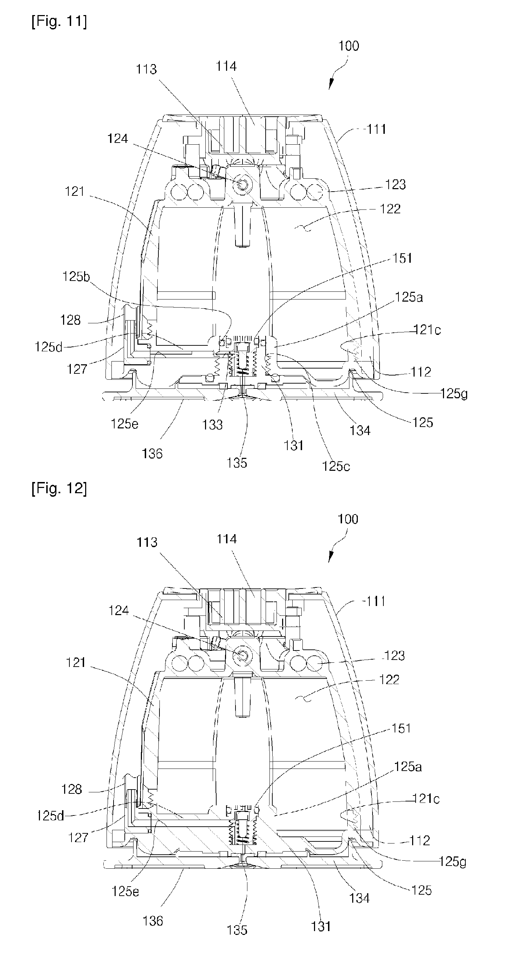

Fig. 11 is a sectional view illustrating a table cleaner according to another embodiment of the present invention.

Referring to Fig. 11, a table cleaner 100 according to another embodiment of the present invention includes a main body 110, a steam generation unit 120, an ejection cleaning unit 130, a support charging unit 140, and a pressure regulation unit 150. The configuration of the table cleaner 100 according to another embodiment of the present invention is partially identical to that of the table cleaner 100 illustrated in Figs. 1 to 10, so different components will solely be described herein.

A generation fastening portion 121c is formed inside the lower portion of the steam generation member 121 on the inner surface of the lower portion of the steam generation space 122 at a location where the lower portion of the steam generation space 122 is sealed by the steam sealing member 125, and a sealing fastening portion 125g is formed on the outer periphery of the steam sealing member 125 to be fastened to the steam generation fastening portion 121c inside the lower portion of the steam generation member 121. The sealing fastening portion 125g can be fastened to and unfastened from the generation fastening portion 121c by user manipulation so that the steam sealing member 125 can be fixed to and released from the steam generation member 121.

That is, in connection with fastening the steam generation member 121 and the steam sealing member 125, the generation fastening portion 121c and the sealing fastening portion 125g are formed on fastening portions, respectively, so that direct fastening is possible.

Fig. 12 is a sectional view illustrating a table cleaner according to still another embodiment of the present invention.

Referring to Fig. 12, a table cleaner 100 according to still another embodiment of the present invention includes a main body 110, a steam generation unit 120, an ejection cleaning unit 130, a support charging unit 140, and a pressure regulation unit 150. The configuration of the table cleaner 100 according to still another embodiment of the present invention is partially identical to that of the table cleaners 100 illustrated in Figs. 1 to 11, so different components will solely be described herein.

A generation fastening portion 121c is formed on the steam generation member 121, and a sealing fastening portion 125g is formed on the steam sealing member 125, so that the lower portion of the steam generation space 122 can be sealed by fastening.

An ejection member 131 is integrally provided at the center of the steam sealing member 125, so that components of the steam sealing member 125 and the ejection member 131 are simplified, thereby reducing the manufacturing cost and decreasing the production cost. In addition, the steam generation space 122 is opened while the steam sealing member 125 is unfastened from the steam generation member 121, and water is then injected. This makes cleaning convenient and removes water, which remains after steam generation, thereby preventing contamination of the interior by water that would otherwise remain.

Although exemplary embodiments of the present invention have been described in the specification and drawings using specific terminology, this is used in general senses to clarify technical contents of the present invention and help understanding thereof, without limiting the scope of the present invention. It is obvious to those skilled in the art, to which the present invention pertains, that, besides the embodiments disclosed herein, other variants can be made based on the technical idea of the present invention.

### Industrial Applicability

The table cleaner according to the present invention has a distance between the location of generation of steam, which is ejected to clean, sterilize, and dry a table, and the location of steam ejection during use so that steam is ejected from a different direction, thereby preventing inflow of steam into heating and charging portions. The table cleaner also has a dual steam control structure and thus has improved safety.

### Reference Signs List

- 100:: table cleaner
- 110:: main body
- 111:: main body case
- 112:: opening
- 113:: storage battery
- 114:: charging connection recess
- 115:: main body handle
- 120:: steam generation unit
- 121:: steam generation member
- 121a:: generation fastening protrusion
- 121b:: generation fastening hole
- 121c:: generation fastening portion
- 122:: steam generation space
- 123:: heating member
- 124:: steam valve
- 125:: steam sealing member
- 125a:: water injection member
- 125b:: water injection hole
- 125c:: injection fastening portion
- 125d:: steam injection member
- 125e:: steam injection channel
- 125f:: sealing fastening protrusion
- 125g:: sealing fastening portion
- 126:: sealing fastening member
- 127:: steam connection member
- 128:: steam supply tube
- 130:: ejection cleaning unit
- 131:: ejection member
- 132:: ejection fastening portion
- 133:: ejection channel
- 134:: cleaning member
- 135:: ejection hole
- 136:: cleaning recess
- 137:: cleaning material
- 140:: support charging unit
- 141:: support member
- 142:: support space
- 143:: support latch
- 144:: charging protrusion
- 145:: power supply switch
- 146:: power supply line
- 150:: pressure regulation unit
- 151:: regulation member
- 152:: regulation space
- 153:: channel connection hole
- 154:: steam inflow hole
- 155:: regulation fastening portion
- 156:: regulation opening/closing member
- 157:: operation elastic member

## Claims

1. A table cleaner, comprising:
a main body case;
a storage battery disposed on an upper portion of the main body case and configured to accumulate power supplied from outside;
a steam generation unit disposed inside the main body case and configured to generate and supply steam by heating water injected through a lower portion spaced from the storage battery by using power supplied from the storage battery; and
an ejection cleaning unit disposed on a lower portion of the main body case and configured to clean a table surface by ejecting steam, which is generated by the steam generation unit, in a downward direction.

2. The table cleaner of claim 1, wherein the steam generation unit comprises:
a steam generation member disposed inside the main body case and provided with a steam generation space having an opened lower portion;
a heating member disposed on an upper portion of the steam generation member and configured to generate steam inside the steam generation member by generating heat using power supplied from the storage battery;
a steam valve disposed on the upper portion of the steam generation member and configured to communicate with the steam generation space and supply the ejection cleaning unit with steam generated in the steam generation space;
a steam sealing member coupled to a lower portion of the steam generation member to seal the steam generation space having the opened lower portion, the steam sealing member having a water injection member at a center, the water injection member protruding to have a water injection hole through which water is injected, the steam sealing member having an injection fastening portion formed inside the water injection hole so that the ejection cleaning unit is fastened to the injection fastening portion, the steam sealing member having a steam injection member having a steam injection channel formed inside to communicate with the water injection hole so that steam, which is injected from the steam valve through one side of the steam generation member, is supplied to the water injection hole; and
a steam connection member disposed on one side of the steam sealing member, one end of the steam connection member being connected to the steam valve through a steam supply tube, the other end being connected to the steam injection channel so that steam, which is injected from the steam valve, is supplied to the ejection cleaning unit.

3. The table cleaner of claim 2, wherein the steam generation unit further comprises:
generation fastening protrusions disposed at a plurality of locations on an outer periphery of the steam generation member, the generation fastening protrusion each having a generation fastening hole formed inside in a through-hole form;
sealing fastening protrusions protruding upwards from a plurality of locations on the steam sealing member while being positioned to contact and fasten to the generation fastening protrusions, respectively; and
sealing fastening members inserted into the generation fastening holes to fasten the generation fastening protrusions and the sealing fastening protrusions and couple the steam generation member and the steam sealing member.

4. The table cleaner of claim 2, wherein the steam generation unit has a generation fastening portion formed on an inner surface of a lower portion of the steam generation member, the steam sealing member being fastened to the generation fastening portion, and a sealing fastening portion is formed on an outer periphery of the steam sealing member, the generation fastening portion being fastened to the sealing fastening portion, so that the steam generation member and the steam sealing member are coupled.

5. The table cleaner of claim 2, wherein the ejection cleaning unit comprises:
an ejection member disposed on a lower portion of the steam generation unit, water being injected through a center of the ejection member, an ejection fastening portion being formed on an outer surface of the ejection member to be fastened to the steam generation unit, an ejection channel being formed through a lateral surface connected to the steam generation unit so that steam is supplied downwards;
a cleaning member disposed on a lower portion of the ejection member, an ejection hole being formed through a center of the cleaning member to communicate with the ejection channel so that steam is ejected, a cleaning recess being formed on a lower surface of the cleaning member; and
a cleaning material disposed on the lower surface of the cleaning member and inserted into the cleaning recess to contact the table surface, and
the ejection member is, when unfastened from the steam generation unit, separated from the main body case so that water is injected into the steam generation unit, and the ejection member is, after injection is finished, fastened to the steam generation unit by the ejection fastening portion so that steam generated by the steam generation unit can be ejected through the ejection hole via the ejection channel.

6. The table cleaner of claim 5, wherein the steam sealing member and the ejection member are integrally formed.

7. The table cleaner of claim 1, further comprising a support charging unit, on which the lower portion of the main body case is seated, or to which the storage battery is coupled and charged.

8. The table cleaner of claim 7, wherein a charging connection recess is formed at a center of an upper portion of the storage battery, the support charging unit being inserted and connected to the charging connection recess to supply power,
the support charging unit comprises:
a support member disposed on one side of the main body case, a support space being formed inside the support member, the main body case being seated in the support space, a support latch being formed towards an upper portion of the support space, the ejection cleaning unit being engaged and supported on the support latch;
a charging protrusion protruding upwards from a center of an inner portion of the support member, the charging protrusion being positioned upwards at a center of the support space so that the main body case is inverted, with the storage battery facing downwards, and is inserted into the charging connection recess and charged; and
a power supply switch disposed on one side of the support member and configured to be turned on/off by external manipulation to control power supply to the charging protrusion, and
the support member is configured so that, when the main body case is seated during use, the ejection cleaning unit remains engaged and supported on the support latch, and, during charging, the main body case is inverted and inserted into the support space to enable the storage battery to be inserted into and supported on the charging protrusion and charged.

9. The table cleaner of claim 1, further comprising a pressure regulation unit disposed between the steam generation space and an ejection location of the ejection cleaning unit and configured to open the ejection cleaning unit and ejects steam to outside, when steam is generated by heating of the heating member and when internal pressure of the steam generation space exceeds a preset threshold, and seal the ejection cleaning unit when the preset threshold is not exceeded.

10. The table cleaner of claim 9, wherein the pressure regulation unit comprises:
a regulation member disposed inside the ejection cleaning unit, the regulation member having a regulation space having one side open in a steam ejection direction of the ejection cleaning unit and the other side sealed towards the steam generation unit, the regulation member having a channel connection hole formed on a lateral surface to communicate so that steam generated by the steam generation unit is supplied, the regulation member having a steam inflow hole formed at a center of the sealed other side of the regulation space to communicate with the steam generation unit so that heated steam flows in, the regulation member having a regulation fastening portion formed on an outer periphery to be fastened to the ejection cleaning unit;
a regulation opening/closing member disposed inside the regulation member and installed inside the regulation space between a location of one side of the steam inflow hole and a steam ejection location of the ejection cleaning unit so as to open/close the steam inflow hole and the steam ejection location of the ejection cleaning unit; and
an operation elastic member disposed between the ejection cleaning unit and the regulation opening/closing member and installed to provide elastic force in such a direction that the regulation opening/closing member seals the steam inflow hole.
